# EUROPEAN PATENT APPLICATION

(11) **EP 4 202 051 A1**
(43) Date of publication of application: **28.06.2023**
(21) Application number: 21383208.2
(22) Date of filing: 23.12.2021
(51) Int. Cl.: C12P 7/54, C12P 7/56, C12M 1/34, C12M 1/00

(54) **A METHOD AND A SYSTEM FOR THE OBTENTION OF HIGH-PURITY VOLATILE FATTY ACIDS**

(71) Applicant: Fundación Centro Gallego de Investigaciones del Agua, 15782 Santiago de Compostela (ES)
(72) Inventor: SILVA TEIRA, Alvaro, 15702 SANTIAGO DE COMPOSTELA (ES); CASERO DIAZ, Tamara, 32500 O CARBALLIÑO (ES)
(74) Representative: Sugrañes, S.L.P.

(57) **Abstract**

A process for obtaining and recovering volatile fatty acids (VFAs) comprising i) supplying a by-product stream from the food industry; ii) subjecting the stream to a hydrolysis operation, if required, in order to solubilize C contained in the stream; iii) subjecting the stream or alternatively the result of the pre-hydrolysis operation to an incomplete fermentation operation, under anaerobic conditions in a reactor, triggering acidogenesis reactions but seeking the inhibition/minimization of methanogenesis reactions; and iv) subjecting a result of the incomplete fermentation operation to cascade separation operations including an operation selected from microfiltration (MF) or ultrafiltration (UF) to remove solids and gross impurities and polymers present in the outlet stream from the reactor; and a subsequent combined operation of extraction/distillation in order to remove water content and to increase the concentration of at least one targeted VFA.

## Description

### Field of the invention

The invention relates to a method and a system for the obtention of volatile fatty acids, particularly for their recovery from a feedstock different from that based on petroleum-oil and natural gas sub-products.

### Background of the invention

Volatile fatty acids, referred to as VFA, are linear short-chain aliphatic mono-carboxylate compounds, such as acetic acid, propionic acid, and butyric acid. Specifically, VFA have two (acetic acid) to six (caproic acid) carbon atoms.

VFA have various applications including green-oils production, polymers synthesis and N removal and are high added value raw materials, reaching at the date values up to 4,000 €/ton or higher, depending on the achieved quality of the product and the type of VFA.

Currently, the production of VFA is 99% based on fossil petroleum-oil and natural gas processing sub-products. However, more sustainable alternatives are desirable, aligned with the in-force decarbonization policies worldwide, especially in Europe.

Among the industrial biotechnologies, the acidogenic fermentation allows the conversion of fermentable organic carbon by bacteria in various products. These comprise in particular carboxylic acids, specifically volatile fatty acids (VFA) and lactic acid, alcohols, dihydrogen and carbon dioxide. When these reactions are promoted in a reactor under anaerobic conditions the acidogenic/acetogenic fermentation is followed by a methanogenic step, thereby producing carbon dioxide and methane as biogas. The inhibition of this methanogenic step in a biological reactor offers thus a possibility to obtain VFA.

The patent document WO 2016/012701 describes a method for the production of organic acids of 1 to 8 carbon atoms from a fermentable biomass. By fermentable biomass is meant an organic substrate, advantageously but not exclusively, non-food, obtained from waste, byproducts and coproducts formed of organic materials, that is to say of the biomass, resulting from human activities, whether they are domestic, industrial, agricultural, forest, aquaculture, agro-industrial or end of the rearing. By way of nonlimiting example, mention is made of organic sludges as substrate, the organic fraction of household refuse, the coproducts from an abattoir, of cellulose or lignocellulose residues originating from the agrifood-industry such as those derived from the transformation of sugar cane (bagasse), sunflower or soybean.

An extraction of VFA is implemented to minimize the presence of inhibitory concentration into carboxylic acids. The method makes it possible to obtain generally VFA at a concentration of between 10 g/L and 20 g/L. Organic acid products comprise between 2 to 4 then primarily carbon atoms. To obtain organic acids from longer chain, in particular organic acids comprising 5 and 6 carbon atoms, secondary fermentation is necessary (chain elongation). This method may require the installation of several reactors arranged in series.

### Summary of the invention

VFA are among the essential intermediates produced when organic waste is treated in anaerobic digestion processes, such as those promoted to produce biogas from fermentable biomass. Those are produced during acidogenesis and acetogenesis stages of the said anaerobic processes. Numerous reactions and microorganisms interact to transform the organic matter firstly into intermediate products, including VFA, and finally into biogas.

Taking advantage of this principle the inventors propose the use of a specific selection of a liquid feedstock, different to that proposed in the state of the art, from which VFA can be obtained in an economically profitable way if
- certain measures are taken to pretreat this feedstock, if necessary,
- proper operational conditions are maintained when the feedstock is subjected to an anaerobic fermentation process, that favor VFA accumulation but inhibits methanogenic reactions which consume the VFA previously obtained, and
- targeted VFA contained in the result of the anaerobic process are selectively separated to obtain at least one marketable VFA based product with similar characteristics than those products with a fossil origin already available in the market:
   ∘ Acetic acid > 70%
   ∘ Propionic > 90%
   ∘ Butyric acid > 90%

It is therefore a first object of the present invention a process for obtaining and recovering volatile fatty acids (VFA), understood as C2-C5 short chain compounds, which comprises
i) supplying a by-product stream from the food industry;
ii) subjecting the stream to a hydrolysis operation, in order to solubilize C contained in the stream, if total or volatile suspended solids (TSS/VSS) in the stream are higher than a predetermined value and/or if fats accounts at least a predetermined percentage of the organic carbon present in the feedstock;
iii) subjecting the stream or alternatively the result of the pre-hydrolysis operation (if required) to an incomplete fermentation operation, under anaerobic conditions in a reactor, triggering acidogenesis reactions but seeking the inhibition/minimization of methanogenesis reactions; and
iv) subjecting a result of the incomplete fermentation operation to cascade separation operations including
   - an operation selected from microfiltration (MF) or ultrafiltration (UF) to remove solids and gross impurities and polymers present in the outlet stream from the fermenter; and
   - subsequent combined operations of extraction/distillation to increase the quality of at least one targeted VFA coming from the MF/UF permeate stream.

In this way, the major amount of water contained in the effluent stream of the reactor can be eliminated and the concentration of the final product, based on a targeted specific VFA, is increased.

With regard to the selected by-product stream from the food industry, the use of streams from the canning or dairy industries has surprisingly been demonstrated to be an effective as well as a more sustainable alternative to non-renewable sources, such as petroleum-oil and natural gas processing sub-products.

More specifically, the organic carbon present in the streams from the fish canning and other specific by-products from milk industries to date with no apparent practical use, have been revealed advantageous for the obtention of VFA.

Particularly, mussels-boiler and tuna-boiler wastewaters can be employed as a feedstock to produce VFA.

Particularly also, a specific dairy by-product, which is a milk permeate, can be employed as a feedstock to produce VFA. In the context of the present invention milk permeate is to be construed as the result of decreasing the amount of water from the raw milk by filtering the feedstock obtaining a concentrated of milk that is used to obtain cheese and others from the reject stream.

These streams, that can be considered by-product stream from the food industry, nowadays considered residual or by-product streams, such as the above exemplified cannery and dairy by-product streams, have been revealed advantageous for the obtention of VFA because of their essential composition, not only because the high concentration of organic carbon present in those streams but also because an equilibrated ratio between this parameter and nutrients. Moreover, the level of salinity of these proposed by-product streams from the food industry have revealed to be adequate for a biological treatment.

In an embodiment of the invention, the specific by-product stream from the food industry selected for obtaining and recovering volatile fatty acids (VFA) complies with the following:

**Table 1: Specific by-product stream parameters of preferred feedstocks from the food industry**

| | |
|---|---|
| Solids concentration | < 2.000 mg TSS/L |
| COD_{T} | 1 < COD < 80 g/L |
| Conductivity | < 35 mS/cm |
| TN | 200 < TN < 4.000 mg/L |
| TP | > 30 mg/L |

The hydrolysis operation, decoupled from the reactions produced downstream inside the reactor, is targeted to increase the availability of soluble carbon in water phase for its conversion to VFA when solids or large polymers are present in the substrate.

It is recommended to apply the hydrolysis step when VSS are higher than 500 mg/L, preferably higher than 1000 mg/L, and/or fats accounts at least the 10% of the organic carbon present in the feedstock.

Anyway, according to an embedment of the invention, further specific measures for preparing the feedstock, here the by-product stream from the food industry, before entering the reactor are the following:
- Setting the pH to 4.5-6.5 to achieve the requirements for promoting an acidic fermentation, for example by directly adding HCI/NaOH of industrial quality, depending on the initial pH of the feedstock.
- Reducing fats concentration to facilitate the contact between biomass and substrate in the reactor, for example by passing the feeding through a dissolved/cavitation air flotation unit if the stream contains more than 1 g/L of fats.

With regards to the incomplete fermentation operation in the reactor, this is proposed to be carried out in such a way that the last stage, methanogenesis, is inhibited and the feeding products of methanogenesis stage, VFA, are accumulated.

In an embodiment of the present invention, the reactor is an upflow expanded bed fermenter with a recirculation to guarantee an optimum upflow velocity (0.15-0.30 m/h) and the correct biomass bed expansion. Preferably, the recirculation is an external recirculation.

The organic carbon degradation requires high-efficient microbial communities. Preferably, the type of biomass to be inoculated should be adapted to fermentation processes rather than methanogenic processes, meaning that a scarcity of methanogens should be present. Microbiota present in the anaerobic biomass hosted in the reactor should be scarce in methanogens. Besides, physically, biomass should have the capability of its aggregation to increase the bulk density. This fact permits staying inside the reactor meanwhile allowing the upflow of the liquid under treatment and promoting biomass/liquid contact. An adapted biomass complying with these conditions can be employed as inoculum to deploy its maximum efficiency in combination with the feedstocks proposed in the present invention.

The adapted biomass is hosted in the bottom portion of the reactor due to the increased density of the bulk biomass respect to the water density. A method for obtaining this adapted biomass, with inactivated methanogens, from a digester/reactor aimed at obtaining biogas, comprises the steps of:
- Extracting anaerobic biomass from either an anaerobic digester from an urban WWTP or from an industrial anaerobic reactor. Both digesters and reactors have been normally addressed to obtain methane-rich biogas.
- Subjecting the extracted anaerobic biomass to an adaptation process carried out in a secondary reactor under acidic levels of pH, whereby the extracted anaerobic biomass acquires the capacity to cause concurrent reactions for VFA production instead of performing the overall anaerobic process all the way to biogas.

The secondary reactor, such as side 200 L capacity reactor having a similar or a same configuration as that described herein after for the reactor to produce VFA, is an upflow reactor including recirculation.

Preferably, the above-mentioned adaptation process comprises an initial adapting cycle treatment essentially consisting of a) a step of feeding a quantity of 3 volumes of cannery/dairy by-product per 1 volume of the initial anaerobic sludge from a digester/reactor. Those conditions will be maintained without entering new feeding for 3-4 days period with the internal recirculation enabled to maintain stirred conditions within the reactor. After this period, b) an inflow rate equivalent to 2-5 days of hydraulic retention time (HRT) to prepare a suspension mixture with the internal recirculation activated is set. In this latter period/stage, pH is controlled by adding HCI/NaOH directly inside the secondary reactor at its bottom part thereof to maintain the pH in the range of 4.0-4.5. By following this strategy, methanogens are expected to be inactivated.

The adaptation process comprises a stabilization step, maintaining the conditions set in the step b) of the initial adapting cycle, at least for 30 days.

The adapted biomass can then be extracted from the secondary reactor.

Later, a new cycle for adapting fresh biomass can be started.

It is worth mentioning that adapted biomass can be saved in the fridge at 0-5 °C for one year at maximum.

Following with the main reactor's features, a heating jacket is installed enwrapping the reactor to ensure the operation at a controlled temperature of 35-37°C. The reactor is operated at atmospheric pressure.

From the microbial point of view, methanogens are normally fed by VFA to produce methane in the complete anaerobic fermentation process. In this specific case, the interest is to stop the fermentation process in acidogenesis to promote VFA accumulation, which are the targeted products. Thus, methanogens' activity must be mostly inhibited in order to reduce the consumption of VFA but improve the yield of the interesting species, VFA. To this aim, operational conditions including temperature, HRT and pH should be carefully cared to enhance the extension of the anaerobic fermentation.

As to the temperature: In an embodiment of the invention, a controlled temperature of 35-37°C inside the reactor is established. To this aim, it is proposed to heat the hydrolysis tank to pre-heat the stream, in case pre-treatment thereof is needed; and equip the reactor with the above-mentioned heating jacket enwrapping the reactor to either supply heat or to minimize heat losses from the reactor to the environment.

As to the hydraulic retention time (HRT): HRT is the ratio between the reactor volume and the feed flow rate. In an embodiment of the invention this parameter is set and maintained by the operator by setting the inlet flow in the reactor since the fermenter volume is a constructive parameter and is not settable. HRT should be set in a range as low as 0.5-5 days. Values in the range of 15-20 days are usually set when operating other technologies and substrates available in the state of the art.

As to the pH: Complete anaerobic reduction of the carbon contained in the wastewater, all the way to methane, should be carried out in a pH range of 6.8-7.2. The aim of the anaerobic fermentation is to promote the organic carbon conversion into VFA, hampering their further conversion to methane. An operation at pH-values below than those above-indicated significantly reduces methanogens' activity, promoting an accumulation of VFA. To this aim, in an embodiment of the invention pH is carefully maintained at pH of 4.5-6.5 inside the reactor.

Thus, pH is to be considered as a crucial variable in a preferred embodiment of the invention. For controlling the pH in the reactor, two points of simultaneously chemicals addition are therefore considered in a preferred variant of the invention:
- A first addition point in a homogenizer tank, to provide a pH-value approaching the value required in the VFA fermentation process.
- A second addition point at the reactor itself, directly adding a chemical specie into the bottom portion of the reactor to obtain a pH-value in the range of 4.5-6.5. The dosage is carried out by a pump when pH-value directly measured in the said reactor is outside the desired range. The pH homogenization in the reactor is advantageously carried out with the turbulence generated by the recirculation stream.

The chemical substance will be either acidic (advised HCI) or alkaline (commonly NaOH), depending on the initial pH of the feeding stream in the points of addition.

It is worth reminding that the presence of acidogenic adapted biomass with scarce presence of methanogens in the microbiota mix allowing the increase of the carbon fermentation extension towards VFA instead of biogas increases the VFA efficiencies.

Further, producing an internal recirculation inside the reactor causes the bed to "expand", that is, the biomass occupies more height within the reactor and makes better use of its volume than in solutions without this recirculation.

In the present case, particularly operating with scarce of methanogens adapted biomass, it was observed that the biomass is not carried along with the treated effluent and remains hosted in the reactor. This means that this low-rate proliferation of the biomass could even resemble an infinite permanence of the biomass in the reactor, only with small and controlled purges, meaning that the solids management costs are extremely low.

Regarding the cascade separation operations, they are preferably performed in a combined train of micro/ultrafiltration followed by extraction/distillation operations. These two steps, are described as follows:
- Filtration operation: Microfiltration (MF) or ultrafiltration (UF) membranes are used for removing macroscopic solids or large polymers which may be pushed out with the liquid effluent stream form the reactor. In this way, a total absence of these substances is warranted.
- Extraction/distillation operations: By means of a specific organic solvent such as ethyl alkanoates, or similar; acetic, propionic and/or butyric acids are dissolved. Water and the organic solvent are not miscible and two liquid easily separable immiscible phases are originated. The solvent portion is majorly recovered by distillation and returned to the extraction operation.

Other features and associated benefits of a process according to the invention are described in the description that will follow.

It is a second object of the present invention a system suitable for obtaining and recovering volatile fatty acids, understood as short chain C2-C5 compounds, from a by-product stream of the food industry, the system comprising:
a) by-product stream pre-treatment means comprising at least one of chemical adaptation means adapted to set a targeted pH within the range 4.5-6.5,
   reducing fats concentration means if the stream contains more than 1 g/L of fats, and
   means for subjecting the stream to a hydrolysis operation, in order to solubilize C contained in the stream, when TSS/VSS concentration is larger than 1,000 mg/L and/or fats' organic carbon > 10% of the overall organic carbon of the feedstock,
b) means to subject the by-product stream, or the pre-treated by-product stream if applicable, to an incomplete fermentation operation, under anaerobic conditions in an upflow reactor, prepared to trigger hydrolysis/acidogenesis reactions but seeking to mostly inhibit methanogenic reactions, wherein the reactor is equipped with:
   means to monitor and control pH conditions inside the reactor
   means to monitor the redox potential
   means for recirculating contents from a middle portion thereof to a bottom portion able to alter the upflow velocity throughout the reactor,
   the system further comprising,
c) means for subjecting the result of the incomplete fermentation operation to cascade separation operations, comprising a first MF or UF separator equipment and an extraction/distillation train to increase the purity up to commercial qualities and the individual separation of the VFA of interest.

### Brief description of the drawings

Figure 1, is a schematic flow diagram of a process for obtaining VFA according to an embodiment of the present invention;
Figure 2, is a schematic flow diagram of a process for obtaining VFA according to another embodiment of the present invention;
Figure 3, is a schematic side view of a reactor for obtaining VFA;
Figure 4, is a schematic top view of the reactor of Figure 2;
Figure 5, shows COD into VFA transformation evolution within 250 days of operation in a reactor during a process according to the invention;
Figure 6, shows individual VFA distribution in the reactor's outlet by fraction; and
Figure 7, shows a schematic flow diagram of an alternative cascade separation operations, according to another embodiment of the present invention.

### Detailed description of the invention

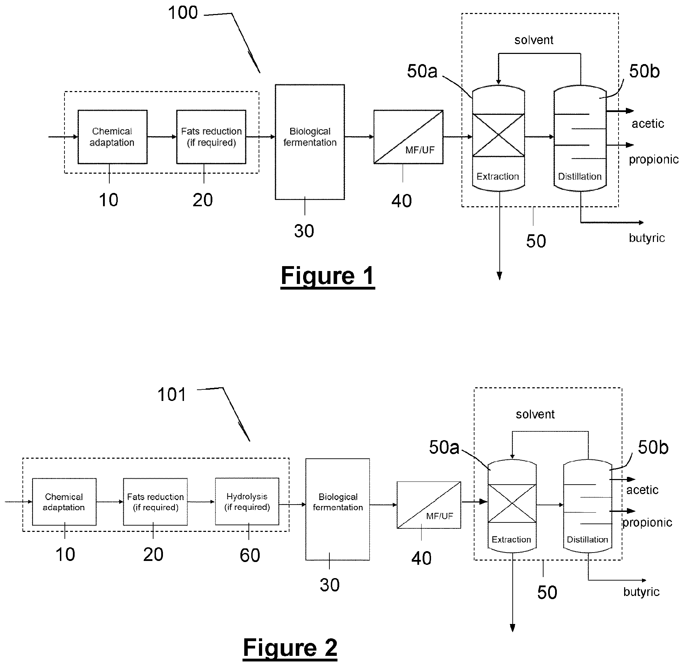

Figure 1 is a schematic flow chart of a first embodiment of a process 100 for obtaining and recovering volatile fatty acids (VFA) according to the invention, specifically suitable for obtaining VFA starting from a by-product stream from the dairy industry, such as the permeate of a raw milk subjected to a membrane separation process. This process 100 comprises the preparing operation of chemical adaptation 10 and the preparing operation of fat reduction 20 of the said stream, if the stream contains more than 1 g/L of fats. The operation of subjecting the obtained pre-treated stream to an incomplete fermentation operation 30, under anaerobic conditions in a reactor, triggering acidogenesis reactions but seeking the inhibition/minimization of methanogenesis reactions; the operation of subjecting a result of the incomplete fermentation operation 30 to separation operation 40, including an operation selected from microfiltration (MF) or ultrafiltration (UF) to remove solids and gross impurities and polymers present in the outlet stream from the fermenter (40); and a final operation of extraction/distillation 50 to isolate a targeted VFA.

With regards to the preparing operations, the operation of chemical adaptation 10 comprises setting the pH to 4.5-6.5 to achieve the requirements for promoting an acidic fermentation, for example by directly adding HCI/NaOH of industrial quality, depending on the initial pH of the feedstock stream and the operation of fat reduction 20 comprises reducing fats concentration to facilitate the contact between biomass and substrate downstream, in the reactor, for example by passing the feeding through a dissolved/cavitation air flotation unit if the stream contains more than 1 g/L of fats.

The operation of subjecting the obtained pre-treated stream to an incomplete fermentation operation 30, under anaerobic conditions, is performed in a reactor 31.

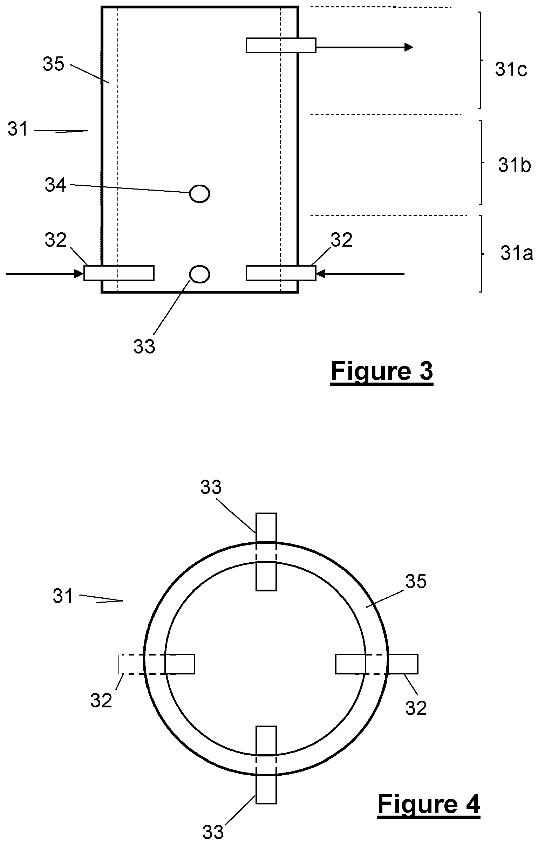

An appropriate reactor 31 is shown in the Figures 3 and 4 and its operation to put into practice the invention is described here below.

The reactor 31 is an upflow expanded bed fermenter with a recirculation to guarantee an optimum upflow velocity (0.15-0.30 m/h) and the correct biomass bed expansion. In the exemplary embodiment, the recirculation is an external recirculation.

The reactor 31, which is cylindrically shaped, is described from the bottom to the top part of the same. Three portions are defined (see Figure 3): a bottom portion 31a comprising the first third of the reactor with respect to its height; a middle portion 31b comprising the second third of the reactor; and an upper portion 31c comprising the remaining third of the reactor 31.

The bottom portion 31a of the reactor 31 is equipped with two sets of different inlet pipelines, each set formed by two pipelines, the said pipelines arranged each at 90° with respect to adjacent ones (see Figure 4) the pipelines of the first and of the second set being arranged alternated. The inlet pipelines of the first set are oppositely faced and arranged at 180° and they are the inlet pipelines to feed the stream to be treated. We will refer to them as feeding inlets 32 hereafter. The inlet pipelines of the second set are oppositely faced and arranged at 180° and they are the inlet pipelines to feed recirculated stream coming from the second third 31b of the reactor 31 to expand the biomass bed. We will refer to them as recirculation inlets 33 hereafter.

In the above-mentioned middle portion 31b of the reactor 31, the reactor 31 is equipped with one outlet pipeline 34 latterly split in two different streams leading to the respective recirculation inlets 33. These two streams are therefore driven to the bottom portion 31a of the reactor 31, entering to it through the associated recirculation inlets 33. Thus, feeding and recycling streams are put in contact internally in the bottom part 31a of the reactor 31 to increase the upflow velocity pulling the flow towards the upper portion 31c of the reactor 31. In this way, the contact between biomass/feedstock can be maintained during a larger distance throughout the height of the reactor, increasing the extension of the carbon fermentation. Along the middle portion 31b of the reactor 31, biomass and effluent are separated due to an increased density of the biomass in comparison to water.

In the upper portion 31c of the reactor 31, once biomass/water are already in separated phases, effluent will be discharged through at least one effluent channel 34 connected to a lung or intermediate storage tank (not illustrated), before being transferred towards the purification/separation equipment.

A heating jacket 35 is provided to the reactor enwrapping the reactor 31 to ensure the operation of the same at a controlled temperature of 35-37°C. Heat energy can be provided by an external water stream with no direct connection with the treatment stream, only for energy interchange purposes. The reactor 31 is operated at atmospheric pressure.

With regards to the subsequent operations, they are aimed at increasing the quality of the collected VFA. The major amount of water contained in the effluent stream from the reactor is eliminated and the concentration of the final individual targeted products will increase.

In the embodiment of the Figure 1 the operations of separation 40 and extraction/distillation 50 are performed in a combined train of micro/ultrafiltration followed by an extraction/distillation step. Microfiltration (MF) or ultrafiltration (UF) membranes are used for removing macroscopic solids or large polymers which may be pushed out with the effluent stream from the reactor. In this way, a total absence of these substances is warranted. The extraction operation 50a is performed by means of a specific organic solvent, such as ethyl alkanoates, or similar to which acetic, propionic and/or butyric acids are dissolved. Water and the organic solvent are not miscible and two liquid easily separable immiscible phases are originated. The solvent is majorly recovered at the distillation operation 50b and returned to the extraction operation 50a.

That is to say, the extraction operation 50a is based on a liquid-liquid extraction. The liquid from the MF/UF unit, aqueous matrix, is put in contact with an organic substance. An increased solubility of the targeted compounds (acetic, propionic, butyric, ...) to the organic substance promoted a net transfer of mass from the aqueous phase to the organic. In the subsequent distillation operation 50b, the organic solvent is evaporated and condensed to be reused in the separation circuit.

Figure 2 is a schematic flow chart of a second embodiment of a process 101 for obtaining and recovering volatile fatty acids (VFA) according to the invention, this time specifically suitable for obtaining VFA starting from a by-product stream from the cannery industry, such as the tuna and mussel boilers' by-product. This process 101 is similar to that of the Figure 1 with the exemption of that an operation of fat reduction 20 of the stream is usually required; and that the preparing operations include the subsequent operation of subjecting the stream to a hydrolysis operation 60, in order to solubilize C contained in the stream, recommended if total or volatile suspended solids (TSS/VSS) in the stream are higher than 1.000 mg/L.

The hydrolysis operation 60 is decoupled from the reactions produced downstream inside the reactor and is targeted to increase the availability of soluble carbon in water phase for its conversion to VFA when solids or large polymers are present in the substrate. The invention envisages different alternatives for the hydrolysis: thermal, chemical or enzymatic can be implemented.

A settable unit in which the operation conditions and the type of hydrolysis, or a combination of them, can be selected. This "multi-hydrolysis unit" can comprise a device which supplies heat for performing a thermal hydrolysis, for example by means of residual heat stream in the same factory or by means of an electric source of heat, such as an electric resistance. The "multi-hydrolysis unit" can also be equipped with dosage means to add the required amount of chemicals and/or enzymes, commonly a dosing pump combined with stirring means to homogenize the concentration of the liquid phase of the treated stream.

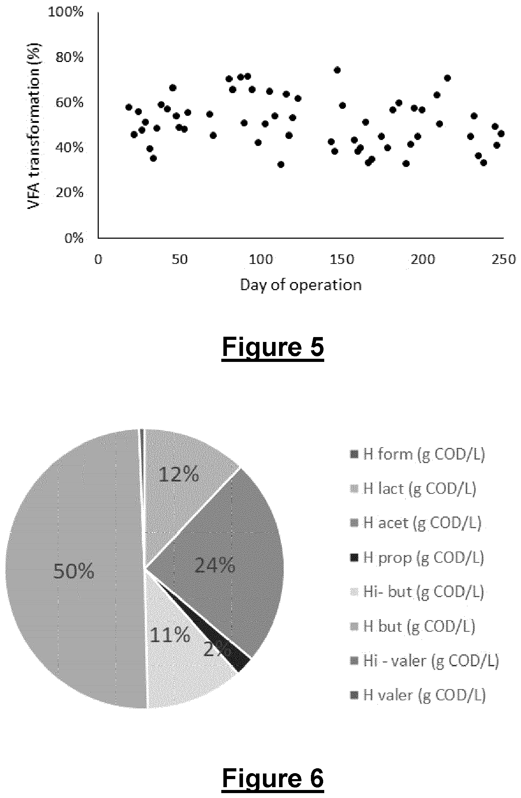

Figures 5 and 6 corresponds to experimental data of the conversion of COD into VFA. In this case, the results were collected using dairy by-products samples at lab-scale. The same dairy by-product employed in Example 1 for feeding a larger-scale prototype had been fed to lab reactors to figure out the extension of the conversion and the VFA sharing in the reactor's outlet. Also, temperature, pH and HRT were equal than that described for the larger-scale prototype. In Figure 5, the observed results indicated a stable conversion of 60%. It was stably observed specially when pH was set at 6.5 and the HRT was established at 2-3 days.

VFA sharing is shown in Figure 6. In this case, it was observed a major distribution of butyric acid (up to 61%) and acetic in the second position with a 24%, when dairy by-product was fed and the above-mentioned experimental conditions were maintained. As it is confirmed in the literature, acids sharing is normally consequence of the carbohydrates/lipids/proteins distribution in the incoming organic carbon matrix employed as feedstock as well as the HRT and pH set in the reactors.

### Example 1: Dairy by-product

Table 2 describes the incoming feeding to the reactor when a dairy by-product was used as feedstock in the prototype.

A totally soluble COD of 50-60 g/L, noticing a total absence of solids, and the total absence of fats are the most important parameters of those which characterizes the incoming feeding. Moreover, nutrients were balanced to the incoming COD making the influent suitable for being treated through an anaerobic treatment. The overall composition of the by-product was very stable within the duration of the whole test. A pH adequation was carried out before entering the reactor to maintain the pH in the reactor in the required limits (4.5-6.5).

**Table 2: Characterization of dairy by-product used as feedstock.**

| Solids concentration | 0 |
|---|---|
| COD_{T} | 50 < COD < 60 g/L |
| Conductivity | 20-30 mS/cm |
| TN | 200 < TN < 250 mg/L |
| TP | > 40 mg/L |

Analytical methods were either contained and published in Standard Methods or their quality was warranted by the manufacturers of the analytical kits (Hach). The VFA composition had been determined with a gas chromatograph (HP C-610) with two online detectors.

Regarding the operational strategy of the reactor, the said reactor was operated with a hydraulic retention time (HRT) of 0.5-5 days and a controlled temperature of 35-37 °C. The recirculation ratio R (Q_{recirculation}/Qᵢₙₗₑₜ) was set at 3-5 within a period of 300 days of operation.

The experimental plan included a pH variation in the range of 4.5 to 6.5 with an HRT fixed at 2-3 days. pH-value was controlled by adding HCI/NaOH.

The reactor was inoculated with biomass from an anaerobic digester adapted to obtain VFA instead of biogas by applying the method proposed in the present invention.

As a result of this operation, total COD fed (50-60 gCOD/L) was stably transformed into VFA in a percentage higher than 60% (30-36 gCOD-VFA/L). The obtained distribution into the different VFA strongly depended on the pH set in each stage of the experimental plan. As a result of setting the pH at 4.5-5.0 the percentage of VFA determined was as follows: HAc: 10-20%; HBut: 80-90% and a negligible fraction of other VFA. When pH was set at 6.0-6.5 acids distribution was of: HAc: 40-50%; HBut: 20-25% and a negligible fraction of other VFA. The above indicated results corresponded to 5-7% (w/w) in all cases, in comparison to the commercial acetic and butyric acids, normally above 60%. This fact meant that a high fraction of water content in the effluent stream from the reactor should be removed to achieve competitive VFA obtained from cannery/diary by-products.

In this sense, a separative train based on an in-series process based on a 1) microfiltration filtration step, coupled by a 2) selective liquid/liquid extraction combined to a 3) distillation step was implemented. Regarding 1), a microfiltration membrane was installed to remove both solids and long-chain polymers operated at < 1 bar of overpressure. Later in 2), a selective dissolvent, belonging to the family of ethyl alkanoates, was employed to extract the carboxylic acids and, as a subsequent step 3), distillation in a same or in a train of distillation towers was performed to majorly recover the solvent to be recycled back to the extraction step and to separate the carboxylic acids among them.

Concerning the combined extraction and distillation separation system, the hydrophilicity indicates the affinity to water of a chemical compound. In this sense, acetic acid was classified as the most hydrophilic compound among the set of compounds of interest, meaning that a separation from water can be complex. As acetic acid was determined as the most hydrophilic among those targeted three (acetic, propionic, butyric), simulations and experimental runs/tests were conducted with this hydrophilic compound.

First, Aspen Hysys simulator was employed to determine the separation efficiencies between the targeted compounds (acetic, propionic and butyric) in the extraction step. Moreover, distillation was also simulated to figure out the solvent recovery in this subsequent phase and the quality of the separated carboxylic acids.

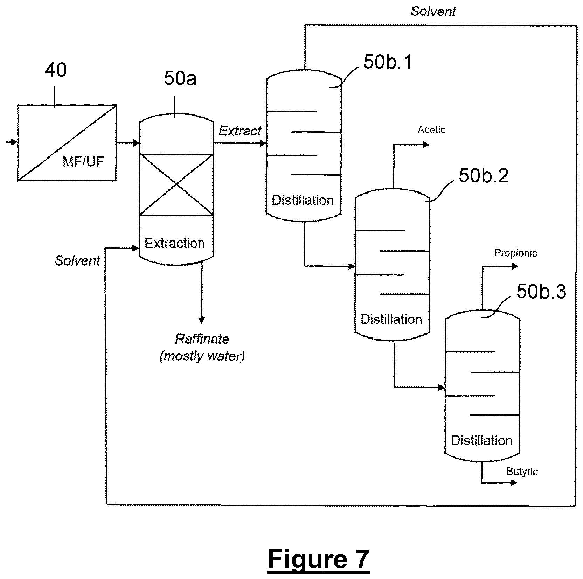

Two different scenarios were simulated depending on the quality of the separated carboxylic acids required. On the one hand, as depicted in Figures 1 and 2, only one distillation tower was set and butyric (appearing at the bottom, less volatile), propionic, acetic and solvent (at the top, most volatile) were separated throughout the height of the distillation tower and depending on their volatility. On the other hand, as depicted in Figure 7, a train of three distillation towers connected in series was considered for obtaining respective high-quality components in the outlets of each distillation tower. The distillation operation is therefore performed in three sub-operations labelled 50b.1, 50b.2 and 50b. 3 in the Figure 7.

Simulation results indicated that acetic acid could be recovered with an efficiency higher than 70% with a solvent / influent relationship of 2 (or higher) in the extraction step.

The simulation indicated that in the case of using one distillation tower 7-8% of solvent could be lost in the distillation step and that the remaining 92-93% could be recycled back and reused in the extraction step. To confirm the simulated results, experimental tests were carried out by employing ethyl octanoate as a solvent and a solvent / influent relationship of 2 and setting us two different facilities, a first one with only one distillation tower as well second one with a train of three distillation towers.

With the operational conditions above explained, an observed efficiency of separation of acetic acid above 70% was obtained in line with the previous simulations. Regarding solvent losses, a ratio below 8% was also determined.

Further, using only one distillation tower, both propionic and butyric were recovered with purities nearly to 90%, and acetic acid above 70%. In contrast, with a train of three distillation towers, as schematically shown in Figure 7, both propionic and butyric were found almost 100% pure and the acetic acid purity was higher than 80%.

### Example 2: Cannery by-product

Table 3 describes the incoming feedstock to the prototype when a cannery by-product was employed as a feedstock. A COD ranged in 8-30 g/L and total suspended solids below 1,000 mg/L are the most important parameters of those which characterizes the incoming feeding. Moreover, nutrients were quite balanced to the incoming COD making the influent suitable for being treated through an anaerobic treatment. In this case, pH was balanced by adding HCI to fulfill an experimental plan similar than the described in the dairy by-product example. In contrast to the above-indicated case, a regulation of the fat's concentration was needed to maintain these compounds in levels enabled to feed the anaerobic reactor.

**Table 3: Characterization of cannery wastewater used as feedstock.**

| Solids concentration | < 1,000 mg/L |
|---|---|
| COD_{T} | 8 < COD < 30 g/L |
| Conductivity | 20-35 mS/cm |
| TN | 0 < TN < 1,200 mg/L |
| TP | > 30 mg/L |

Employed analytical methods were the same than those used in the dairy by-product influent.

Regarding the experimental plan, the reactor was operated with a hydraulic retention time (HRT) of 0.5-5 days and a controlled temperature of 35-37 °C. The recirculation ratio R (Q_{recirculation}/Qᵢₙₗₑₜ) was set at 3-5 within a period of 150 days of operation. pH was maintained in the range of 4.5 to 6.5 and was controlled by adding HCI/NaOH.

The reactor was inoculated with biomass from an anaerobic digester adapted to obtain VFA instead of biogas by applying the method proposed in the present invention.

COD_{T}/COD_{S} and TN in the feed were variable 8-30 g/L and 600-1,200 mgN/L, respectively. COD was stably transformed into VFA in a percentage up to 60%. The obtained distribution into the different VFA strongly depended on the pH. As a result of setting the pH at 6.0-6.5 the percentage of VFA determined was mostly HAc 70-85% and negligible fractions of the remaining VFA.

Regarding the separation, a similar procedure than that above described for the dairy case was performed, including simulation and experimental tests. In this case, the cannery matrix was more complex than the matrix of the dairy case. Results observed for the separation were similar than those observed for the dairy case. Nevertheless, experimental results indicated a separation of about 60% of the acetic acid and solvent losses up to 10%. These lower performances are ascribable to a higher complexity of the matrix with a much higher level of salinity.

Regarding propionic and butyric acids, the observed results indicated a separation performance above 80% with solvent losses up to 10-11 % when only one distillation tower was employed.

## Claims

1. A process for obtaining and recovering volatile fatty acids (VFA), understood as C2-C5 short chain compounds, which comprises
i) supplying a by-product stream from the food industry;
ii) subjecting the by-product stream to a hydrolysis operation, in order to solubilize C contained in the stream, if total or volatile suspended solids (TSS/VSS) in the stream are higher than 500 mg/L and/or if fats accounts at least the 10% of the organic carbon present in the stream;
iii) subjecting the by-product stream or alternatively the result of the pre-hydrolysis operation to an incomplete fermentation operation, under anaerobic conditions in a reactor, triggering acidogenesis reactions but seeking the inhibition/minimization of methanogenesis reactions; and
iv) subjecting a result of the incomplete fermentation operation to cascade separation operations including
- an operation selected from microfiltration (MF) or ultrafiltration (UF) to remove solids and gross impurities and polymers present in the outlet stream from the reactor; and
- a subsequent combined operation of extraction/distillation in order to remove water content and to increase the concentration of at least one targeted VFA.

2. The process according to the claim 1, wherein the by-product stream from the food industry is characterized as follows:
| Solids concentration | < 2.000 mg TSS/L |
|---|---|
| COD_{T} | 1 < COD < 80 g/L |
| Conductivity | < 35 mS/cm |
| TN | 200 < TN < 4.000 mg/L |
| TP | > 30 mg/L |

3. The process according to the claim 2, **characterized in that** the by-product stream from the food industry is one of a stream from the fish canning industries, preferably mussels-boiler wastewater or tuna-boiler wastewater, and wastewater stream from dairy industries, preferably a raw milk permeate.

4. The process according to anyone of claims 1 to 3, **characterized in that** microfiltration (MF) is selected in iv) and **in that** a microfiltration membrane with a cut-off size ranged from 0.1 to 10 µm hosted in a respective membrane housing is used, wherein filtration is operated at a pressure < 1 bar over the atmospheric.

5. The process according to anyone of the previous claims, **characterized in that** the extraction/distillation operations comprises extracting VFAs by dissolving the VFAs contained in the treated stream with an organic solvent not miscible with water; and separately recovering acetic/propionic/butyric acids via a distillation process, the recovered solvent being reused for a subsequent extraction operation.

6. The process according to the claim 5, **characterized in that** for the distillation operation a single distillation tower is used wherein butyric, propionic, acetic and solvent in this volatility order are obtained in respective levels of the said distillation tower.

7. The process according to the claim 5, **characterized in that** for the distillation operation a set of three distillation towers connected in series is used wherein acetic, propionic and butyric in this volatility order are obtained in the respective distillation towers.

8. The process according to anyone of claims 1 to 7, **characterized in that** it comprises a fat-content preparing operation of the by-product stream which is performed upstream of the iii) incomplete fermentation operation, to reduce fats concentration of the by-product stream if the said by-product stream contains more than 1 g/L of fats, to facilitate the contact between biomass and substrate in the reactor.

9. The process according to the claim 8, **characterized in that** the reduction in fats concentration is carried out by passing the by-product stream through a dissolved/cavitation air flotation unit (DAF/CAF).

10. The process according to anyone of claims 1 to 9, **characterized in that** it comprises a pH-regulation preparing operation of the by-product stream which is performed upstream of the iii) incomplete fermentation operation, preferably, if applicable, upstream of a fat-content preparing operation, which consist in setting the pH to 4.5-6.5 to achieve the requirements for promoting an acidic fermentation.

11. The process according to anyone of claims 1 to 10, **characterized in that** the reactor is an upflow expanded bed fermenter operated at atmospheric pressure equipped with
- feeding inlets at a bottom portion thereof, to feed as an influent the stream to be subjected to an incomplete fermentation operation;
- recirculation means to feed at a bottom portion thereof recirculated stream coming from a middle portion of the reactor;
- an effluent channel at an upper portion thereof to discharge an effluent treated stream;
wherein the reactor is operated
- at a controlled temperature of 35-37°C;
- fulfilling an objective hydraulic retention time (HRT) in a range of 0.5-5 days;
- at a pH conditions of 4.5-6.5 inside the reactor.

12. The process according to claim 11, **characterized in that** the biomass contained in the reactor is an adapted biomass with inactivated methanogens, inhibiting the biomass ability of transforming the VFA into methane.

13. A system for obtaining and recovering volatile fatty acids, understood as short chain C2-C5 compounds, from a by-product stream of the food industry, the system comprising:
a) by-product stream pre-treatment means comprising at least one of chemical adaptation means adapted to set a targeted pH within the range 4.5-6.5, reducing fats concentration means if the stream contains more than 1 g/L of fats, and means for subjecting the stream to a hydrolysis operation, in order to solubilize C contained in the stream, when TSS/VSS concentration is larger than 500 mg/L,
b) means to subject the by-product stream, or the pre-treated by-product stream if applicable, to an incomplete fermentation operation, under anaerobic conditions in an upflow reactor, prepared to trigger hydrolysis/acidogenesis reactions but seeking to mostly inhibit methanogenic reactions, wherein the reactor is equipped with:
means to monitor and control pH conditions inside the reactor
means to monitor the redox potential
means for recirculating contents from a middle portion thereof to a bottom portion able to alter the upflow velocity throughout the reactor,
the system further comprising,
c) means for subjecting the result of the incomplete fermentation operation to cascade separation operations, comprising a first MF or UF separator equipment and an extraction/distillation train to increase the purity up to commercial qualities and produce the individual separation of the VFA of interest.

14. The use of a by-product stream from the food industry selected from a stream from the fish canning industries, preferably mussels-boiler wastewater or tuna-boiler wastewater, and wastewater stream from dairy industries, preferably a raw milk permeate, to obtain volatile fatty acids (VFA), understood as C2-C5 short chain compounds, implying directly subjecting the said by-product stream or alternatively the result of subjecting the said by-product stream to at least one of a chemical adaptation, fat removal or hydrolysis operations, to a subsequent incomplete fermentation operation under anaerobic conditions in a reactor, triggering acidogenesis reactions but seeking the inhibition/minimization of methanogenesis reactions.
